# EUROPÄISCHE PATENTSCHRIFT

(11) **EP 0 607 826 B1**
(45) Veröffentlichungstag und Bekanntmachung des Hinweises auf die Patenterteilung: **07.05.1997**
(21) Anmeldenummer: 94100207.3
(22) Anmeldetag: 07.01.1994
(51) Int. Cl.: C07C 63/70

(54) **Fluor-trifluormethylbenzoesäure-Derivate**
Fluoro-trifluoromethylbenzoic acid derivatives
Dérivés d'acide fluoro-trifluorométhylbenzoique

(30) Priorität: 19.01.1993 DE 4301245
(43) Veröffentlichungstag der Anmeldung: 27.07.1994
(73) Patentinhaber: BAYER AG, 51368 Leverkusen (DE)
(72) Erfinder: Marhold, Albrecht, Dr., D-51373 Leverkusen (DE); Andres, Peter, Dr., D-42799 Leichlingen (DE)

(56) Entgegenhaltungen:
- EP-A- 0 302 525

## Beschreibung

Die vorliegende Erfindung betrifft neue Fluor-trifluormethylbenzoesäure-Derivate, neue Zwischenprodukte sowie Verfahren zu deren Herstellung.

Gemäß EP-A 302 525 werden bromhaltige Benzolderivate mit Lithiumisopropylamid und CO₂ zu bromhaltigen Benzoesäurederivaten und bromhaltige Benzolderivate mit Butyllithium zu bromfreien Benzoesäurederivaten umgesetzt. Gemäß Synlett (1990), 747-778 werden Benzolderivate mit maximal zwei Substituenten mit Gemischen aus Butyllithium und anderen Basen umgesetzt. Beim Einsatz von Butyllithium wurden unselektive Reaktionen beobachtet.

Die neuen Fluor-trifluormethylbenzoesäure-Derivate entsprechen der allgemeinen Formel (I) 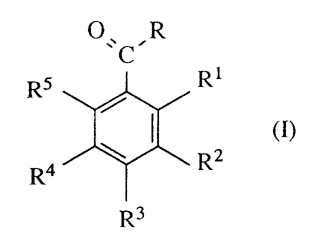 worin
- R: für Hydroxy, -OM, Halogen oder C₁-C₄-Alkoxy steht,
wobei M für ein Alkalimetall, vorzugsweise für Lithium, Natrium oder Kalium steht, und entweder
- a): R¹ für F, R² für F, R³ für H, R⁴ für H und R⁵ für CF₃
- oder b): R¹ für F, R² für CF₃, R³ für F, R⁴ für H und R⁵ für H
- oder c): R¹ für F, R² für CF₃, R³ für H, R⁴ für H und R⁵ für F
- oder d): R¹ für F, R² für F, R³ für CF₃, R⁴ für H und R⁵ für H
- oder e): R¹ für F, R² für H, R³ für CF₃, R⁴ für H und R⁵ für F
- oder f): R¹ für F, R² für F, R³ für F, R⁴ für H und R⁵ für CF₃
- oder g): R¹ für F, R² für CF₃, R³ für F, R⁴ für H und R⁵ für F
- oder h): R¹ für F, R² für F, R³ für H, R⁴ für CF₃ und R⁵ für F
- oder i): R¹ für F, R² für F, R³ für F, R⁴ für CF₃ und R⁵ für H
- oder k): R¹ für F, R² für F, R³ für CF₃, R⁴ für F und R⁵ für H
- oder l): R¹ für F, R² für F, R³ für CF₃, R⁴ für H und R⁵ für F
- oder m): R¹ für F, R² für F, R³ für F, R⁴ für F und R⁵ für CF₃
steht.

Die neuen Fluor-trifluormethylbenzoesäure-Derivate der Formel (I), mit R = Hydroxy, können nach einem Verfahren hergestellt werden, das dadurch gekennzeichnet ist, daß man Fluor-trifluormethylbenzole der Formel (IV) 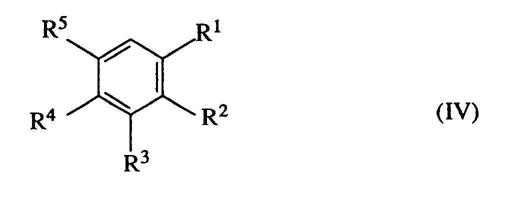 worin
entweder
- a): R¹ für F, R² für F, R³ für H, R⁴ für H und R⁵ für CF₃
- oder b): R¹ für F, R² für CF₃, R³ für F, R⁴ für H und R⁵ für H
- oder c): R¹ für F, R² für CF₃, R³ für H, R⁴ für H und R⁵ für F
- oder d): R¹ für F, R² für F, R³ für CF₃, R⁴ für H und R⁵ für H
- oder e): R¹ für F, R² für H, R³ für CF₃, R⁴ für H und R⁵ für F
- oder f): R¹ für F, R² für F, R³ für F, R⁴ für H und R⁵ für CF₃
- oder g): R¹ für F, R² für CF₃, R³ für F, R⁴ für H und R⁵ für F
- oder h): R¹ für F, R² für F, R³ für H, R⁴ für CF₃ und R⁵ für F
- oder i): R¹ für F, R² für F, R³ für F, R⁴ für CF₃ und R⁵ für H
- oder k): R¹ für F, R² für F, R³ für CF₃, R⁴ für F und R⁵ für H
- oder l): R¹ für F, R² für F, R³ für CF₃, R⁴ für H und R⁵ für F
- oder m): R¹ für F, R² für F, R³ für F, R⁴ für F und R⁵ für CF₃
steht,
mit einem Alkylmetallat, gegebenenfalls in Gegenwart eines Verdünnungsmittels bei einer Temperatur von -150 bis 30°C, vorzugsweise von -100 bis -20°C, umsetzt, so metallierte Fluor-trifluormethyl-benzol-Derivate der Formel (II) erhält 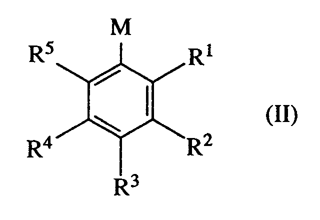 worin
- M: für ein Alkalimetall, vorzugsweise für Lithium, Natrium oder Kalium steht,
und entweder
- a): R¹ für F, R² für F, R³ für H, R⁴ für H und R⁵ für CF₃
- oder b): R¹ für F, R² für CF₃, R³ für F, R⁴ für H und R⁵ für H
- oder c): R¹ für F, R² für CF₃, R³ für H, R⁴ für H und R⁵ für F
- oder d): R¹ für F, R² für F, R³ für CF₃, R⁴ für H und R⁵ für H
- oder e): R¹ für F, R² für H, R³ für CF₃, R⁴ für H und R⁵ für F
- oder f): R¹ für F, R² für F, R³ für F, R⁴ für H und R⁵ für CF₃
- oder g): R¹ für F, R² für CF₃, R³ für F, R⁴ für H und R⁵ für F
- oder h): R¹ für F, R² für F, R³ für H, R⁴ für CF₃ und R⁵ für F
- oder i): R¹ für F, R² für F, R³ für F, R⁴ für CF₃ und R⁵ für H
- oder k): R¹ für F, R² für F, R³ für CF₃, R⁴ für F und R⁵ für H
- oder l): R¹ für F, R² für F, R³ für CF₃, R⁴ für H und R⁵ für F
- oder m): R¹ für F, R² für F, R³ für F, R⁴ für F und R⁵ für CF₃
steht,
die man mit Kohlendioxid, gegebenenfall in Gegenwart eines Verdünnungsmittels bei einer Temperatur von - 150 bis 50°C umsetzt, vorzugsweise von -80 bis 30°C und anschließend die gebildeten Alkalicarboxylate der Formel (I) mit R = OM mit einer Säure wie beispielsweise Salzsäure umsetzt.

Die neuen Fluor-trifluormethylbenzoesäure-Derivate der Formel (I), worin R für Halogen steht, können in an sich bekannter Weise hergestellt werden, indem man Verbindungen der Formel (I) , worin R für Hydroxy oder -OM steht, mit einem Halogenierungsmittel wie z.B. einem Schwefel- oder Phosphorhalogenid gegebenenfalls in Gegenwart eines Vedünnungsmittels, bei einer Temperatur von 0 bis 160°C, vorzugsweise von 60 bis 120°C, umsetzt.

Die neuen Verbindungen der Formel (I), worin R für C₁-C₄-Alkoxy steht, können in an sich bekannter Weise hergestellt werden, indem man Verbindungen der Formel (I), worin R für Hydroxy oder Halogen steht, mit einem Alkohol der Formel (III)

R⁶-OH (III)

worin
- R⁶: für C₁-C₄-Alkyl steht,
gegebenenfalls in Gegenwart eines Verdünnungsmittels und gegebenenfalls in Gegenwart eines Säurebindemittels, bei einer Temperatur von 20 bis 160°C, vorzugsweise von 50 bis 120°C, umsetzt.

Die metallierten Verbindungen der Formel (II) sind neu und ebenfalls Gegenstand der vorliegenden Erfindung.

Als Alkylmetallate kommen z.B. Methyllithium, n-Butyllithium, -natrium oder -kalium, tert.-Butyllithium, -natrium oder -kalium, vorzugsweise n-Butyllithium, in Frage. Die Verbindungen der Formel (IV) sind in der organischen Chemie allgemein bekannte Verbindungen.

Stellt man beispielsweise zunächst in 6-Stellung lithiiertes 1,3-Difluor-2-trifluormethylbenzol her, setzt dieses mit Kohlendioxid um und hydrolysiert anschließend mit Salzsäure, so läßt sich der zweite Teil des erfindungsgemäßen Verfahrens zur Herstellung der Verbindungen (I) mit R = Hydroxy durch folgendes Formelschema darstellen:

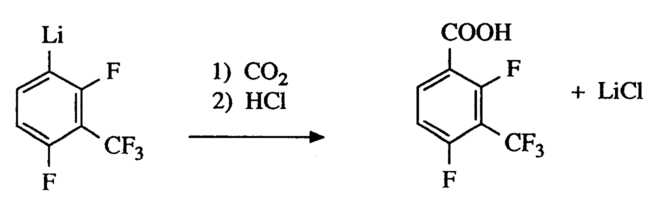

Die Umsetzung der metallierten Fluor-trifluorbenzol-Derivate kann sowohl mit gasförmigem als auch mit festem Kohlendioxid erfolgen. Vorzugsweise wird gasförmiges Kohlendioxid unter Atmosphärendruck in die Reaktionslösung eingeleitet.

Das erfindungsgemäße Verfahren zur Herstellung der Verbindungen (I) mit R = Hydroxy wird vorzugsweise in Gegenwart eines Verdünnungsmittels durchgeführt. Dabei kommen prinzipiell folgende Verdünnungsmittel in Frage: niedere Kohlenwasserstoffe, Ether, oder Gemische aus beiden.

Vorzugsweise verwendet man Pentan, Hexan, Heptan, Diethylether, Tetrahydrofuran als Verdünnungsmittel.

Die Temperatur kann bei dem erfindungsgemäßen Verfahren zur Herstellung der Verbindungen (I) mit R = Hydroxy in einem weiten Bereich variiert werden. Im allgemeinen arbeitet man bei einer Temperatur von -150 bis 50°C, vorzugsweise von -80 bis 30°C.

Zur Durchführung des erfindungsgemäßen Verfahrens zur Herstellung der Verbindungen (I) mit R = Hydroxy geht man zweckmäßigerweise so vor, daß man in eine Lösung der Verbindung (II) in einem Gemisch aus Tetrahydrofuran (THF), Diethylether und Hexan bei einer Temperatur von -80 bis -20°C gasförmiges CO₂ einleitet, in einer Menge von 0,9 bis 10 Mol, vorzugsweise 2,0 bis 6,0 Mol, CO₂ bezogen auf 1 Mol Verbindung (II).

Anschließend wird mit 1 bis 1,5 Äquivalenten Salzsäure hydrolysiert. Die Aufarbeitung erfolgt beispielsweise wäßrig-extraktiv, indem man die Phasen trennt, mit Diethylether extrahiert, anschließend mit Salzsäure und Wasser wäscht, die organische Phase über Natriumsulfat trocknet und einengt.

Verwendet man beispielsweise 2,4-Difluor-3-trifluormethylbenzoesäure als Ausgangsverbindung und setzt diese mit Thionylchlorid um, so läßt sich das erfindungsgemäßen Verfahren zur Herstellung der Verbindungen (I) mit R = Halogen durch folgendes Formelschema darstellen:

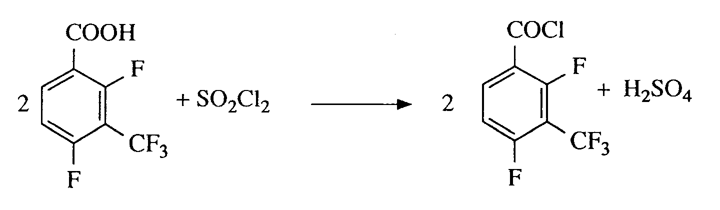

Das erfindungsgemäße Verfahren zur Herstellung der Verbindungen (I) mit R = Halogen wird vorzugsweise ohne Zusatz eines Verdünnungsmittels durchgeführt.

Die Temperatur kann bei der Herstellung der Verbindungen (I) mit R = Halogen nach dem erfindungsgemäßen Verfahren in einen weiten Bereich variiert werden. Im allgemeinen arbeitet man bei einer Temperatur von 0 bis 160°C, vorzugsweise von 60 bis 120°C.

Zur Durchführung des erfindungsgemäßen Verfahrens zur Herstellung der Verbindungen (I) mit R = Halogen geht man im allgemeinen so vor, daß man die Verbindung (I) mit R = Hydroxy vorlegt und unter Feuchtigkeitsauschluß bei einer Temperatur von vorzugsweise 0°C das Halogenierungsmittel, beispielsweise Thionylchlorid in einer Menge von 1-3 Mol, vorzugsweise 2 Mol, bezogen auf 1 Mol Verbindung (I) mit R = OH, zutropft. Anschließend rührt man bis zum Ende der HCl-Entwicklung unter Rückfluß (60-120°C), destilliert überschüssiges SOCl₂ ab und destilliert dann das Produkt.

Verwendet man beispielsweise 2,4-Difluor-3-trifluormethylbenzoesäure und Methanol als Ausgangsverbindungen, so läßt sich das erfindungsgemäße Verfahren zur Herstellung der Verbindungen (I) mit R = C₁-C₄-Alkoxy durch folgendes Formelschema darstellen:

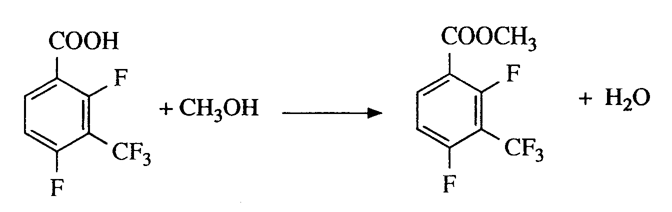

Das erfindungsgemäße Verfahren zur Herstellung der Verbindungen (I) mit R = C₁-C₄-Alkoxy wird vorzugsweise in Abwesenheit eines Verdünnungsmittels durchgeführt. Als Verdünnungsmittel komen aber prinzipiell niedere chlorierte Kohlenwasserstoffe in Frage, wobei Chloroform bevorzugt ist.

Die Temperatur kann bei der Durchführung des erfindungsgemäßen Verfahrens zur Herstellung der Verbindungen (I) mit R = C₁-C₄-Alkoxy in einem weiten Bereich variiert werden. Im allgemeinen arbeitet man bei Temperaturen von 20 bis 160°C, vorzugsweise von 50 bis 120°C.

Zur Durchführung des erfindungsgemäßen Verfahrens zur Herstellung der Verbindungen (1) mit R = C₁-C₄-Alkoxy geht man im allgemeinen so vor, daß man ein Gemisch aus 1 Mol der Verbindung (1) mit R = Hydroxy und 5 Mol des Alkohols der Formel (III) vorlegt, mit 0,2 ml konz. Schwefelsäure versetzt und 5 Stunden unter Rückfluß und Feuchtigkeitsausschluß rührt. Dann wird der Hauptteil des überschüssigen Alkohols abdestilliert und der Rückstand wässrig-extraktiv aufgearbeitet. Der Ester kann zur Reinigung destilliert werden.

Verwendet man beispielsweise 2,4-Difluor-3-trifluormethylbenzoesäurechlorid und Methanol als Ausgangsverbindungen, so läßt sich das erfindungsgemäße Verfahren zur Herstellung der Verbindungen (I) mit R = C₁-C₄-Alkoxy durch folgendes Formelschema darstellen:

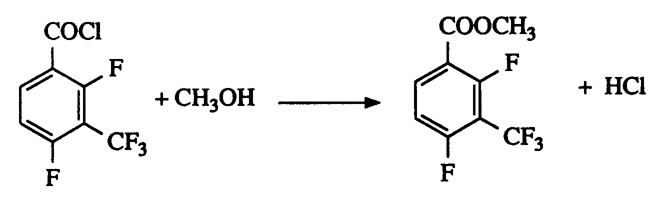

Das erfindungsgemäße Verfahren zur Herstellung der Verbindungen (I) mit R = C₁-C₄-Alkoxy wird entweder ohne Zusatz eines Verdünnungsmittels oder in Gegenwart eines Vedünnungsmittels durchgeführt, wobei als Verdünnungsmittel prinzipiell Ether und chlorierte niedere Kohlenwasserstoffe in Frage kommen.

Ein bevorzugtes Verdünnungsmittel ist Diethylether.

Das erfindungsgemäße Verfahren zur Herstellung der Verbindungen (I) mit R = C₁-C₄-Alkoxy wird gegebenenfalls in Gegenwart eines säurebindenden Mittels durchgeführt. Als säurebindende Mittel kommen beispielsweise tertiäre Amine wie Triethylamin in Frage.

Zur Durchführung des erfindungsgemäßen Verfahrens zur Herstellung der Verbindungen (I) mit R = C₁-C₄-Alkoxy geht man im allgemeinen so vor, daß man zunächst die Verbindung (I) mit R = Halogen in Ether löst und zu dieser Lösung in Gegenwart von NEt₃ als säurebindendem Mittel bei einer Temperatur von vorzugsweise 20-50°C den Alkohol der Formel (III) in einer Menge von 1 Mol, bezogen auf 1 Mol, zutropft. Anschließend wird der Feststoff abgesaugt, das Filtrat wässrig-extraktiv aufgearbeitet und das Produkt gegebenenfalls durch Destillation isoliert.

Verwendet man beispielsweise 1,3-Difluor-2-trifluormethylbenzol und n-Butyllithium als Ausgangsverbindungen, so läßt sich das erfindungsgemäße Verfahren zur Herstellung der Verbindungen der Formel (II) durch folgendes Formelschema darstellen:

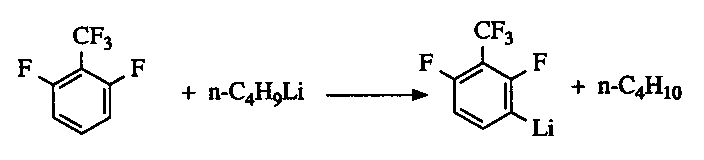

Das erfindungsgemäße Verfahren zur Herstellung der Verbindungen (II) wird vorzugsweise in Gegenwart eines Vedünnungsmittels durchgeführt. Als Verdünnungsmittel komen dabei prinzipiell in Frage: niedere Kohlenwasserstoffe, Ether oder Gemische aus beiden.

Vorzugsweise verwendet man Diethylether, Tetrahydrofuran, Hexan oder deren Gemische.

Das erfindungsgemäße Verfahren zur Herstellung der Verbindungen (II) wird im allgemeinen bei Temperaturen von -150 bis 30°C, vorzugsweise von -100 bis -20°C, besonders bevorzugt -80 bis -50°C, durchgeführt.

Vorzugsweise wird das erfindungsgemäße Verfahren zur Herstellung der Verbindungen (II) unter einer Inertgasatmosphäre durchgeführt, wobei als Inertgase beispielsweise Argon oder Stickstoff in Frage kommen.

Vorzugsweise geht man bei der Herstellung der Verbindungen (II) so vor, daß man das Fluor-trifluormethylbenzol der Formel (IV) in einem 1:1-Gemisch aus THF und Et₂O löst und zu dieser Lösung unter einer Inertgasatmosphäre bei einer Temperatur von vorzugsweise -80 bis -30°C die metallorganische Verbindung, gelöst in Hexan, zutropft.

Die Verbindungen der Formel (II) können durch Entfernen des Lösungsmittels (abdestillieren) isoliert werden.

Die erfindungsgemäßen Fluor-trifluormethylbenzoesäure-Derivate der Formel (I) können beispielsweise verwendet werden zur Herstellung von antibakteriellen Mitteln und Futterzusatzstoffen.

So kann man beispielsweise
1) 2,4-Difluor-3-trifluormethylbenzoesäure durch Umsetzung mit einem Halogenierungsmittel wie Thionylchlorid in 2,4-Difluormethylbenzoesäurechlorid überführen, welches
2) mit Malonsäurediethylester zum (2,4-Difluor-3-trifluormethylbenzoyl)-malonsäurediethylester umgesetzt wird, anschließend
3) die gemäß Schritt 2) erhaltene Verbindung durch Verseifung und Decarboxylierung in den (2,4-Difluor-3-trifluormethylbenzoyl)essigsäureethylester überführt wird, welcher
4) durch Reaktion mit Orthoameisensäureethylester zum 3-Ethoxy-2-(2,4-difluor-3-trifluormethylbenzoyl)-acrylsäureethylester umgesetzt wird,
5) die gemäß Schritt 4) erhaltene Verbindung mit Cyclopropylamin zum 3-Cyclopropylamino-2-(2,4-difluor-4-trifluormethylbenzoyl)-acrylsäureethylester umgesetzt wird,
6) die gemäß Schritt 5) erhaltene Verbindung durch Abspaltung von HF in das entsprechende Chinoloncarbonsäureester-Derivat umgewandelt wird, welches
7) durch Verseifung zum entsprechenden Chinoloncarbonsäure-Derivat umgesetzt wird.

In entsprechender Art und Weise können aus den erfindungsgemäßen Verbindungen der Formel (I) weitere Chinoloncarbonsäure-Derivate hergestellt werden.

Die oben beschriebene Reaktionsfolge kann beispielhaft durch folgendes Formelschema dargestellt werden:

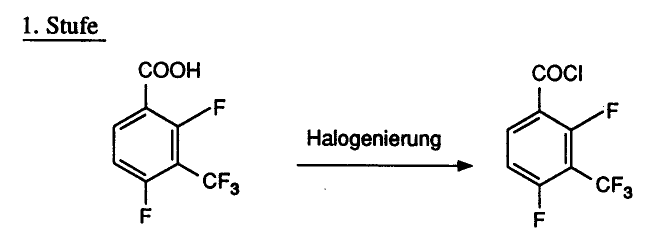

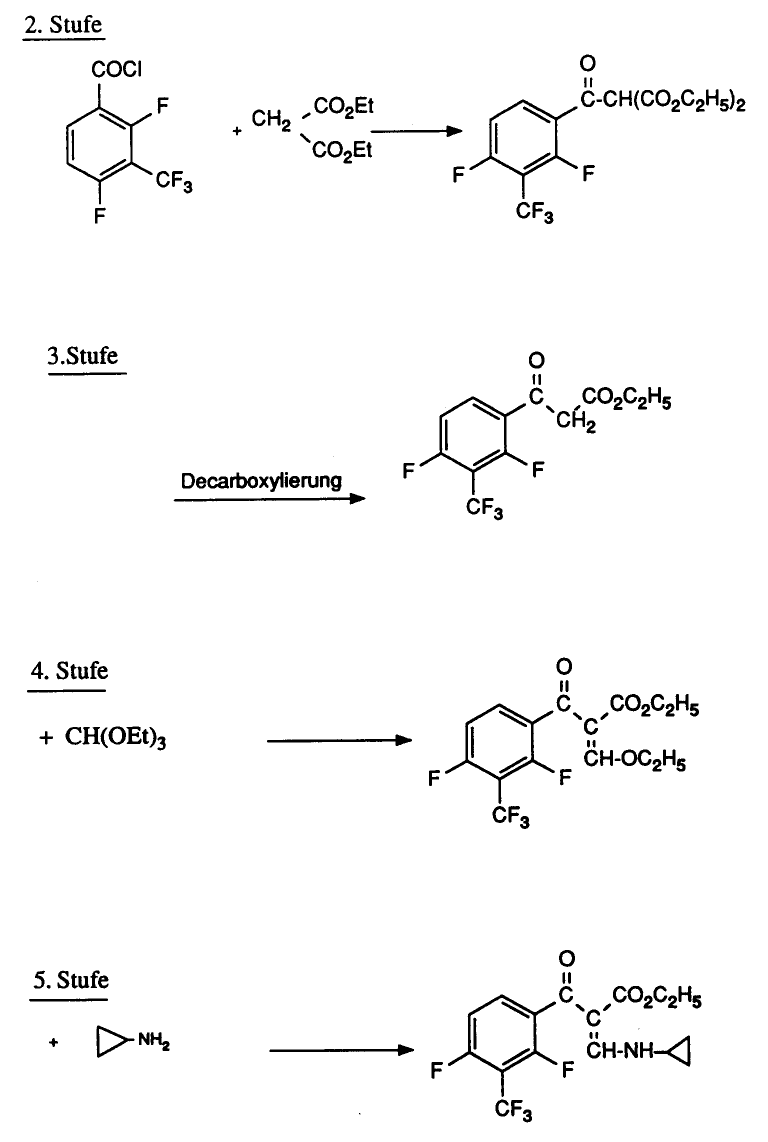

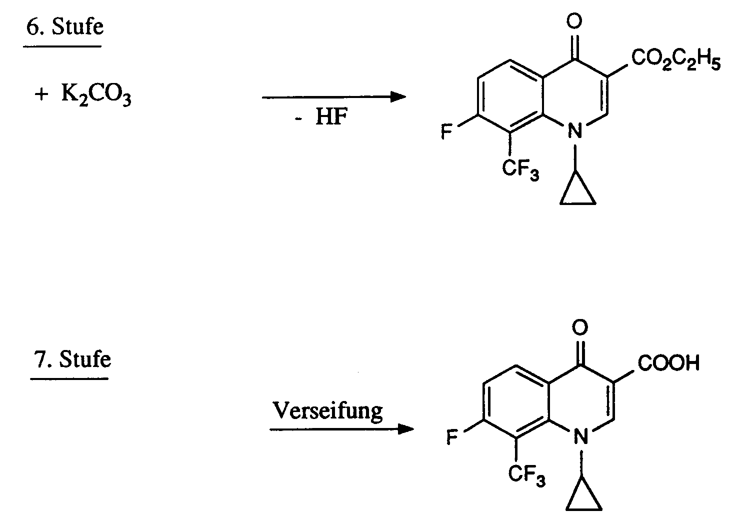

Die in der Stufe 7) erhaltenen Chinoloncarbonsäure-Derivate entsprechen der allgemeinen Formel (V) 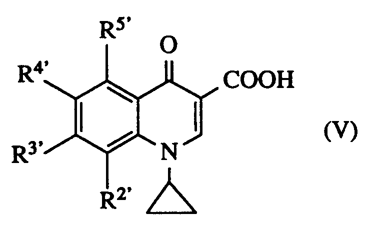 worin
entweder
n) R^{2'} für CF₃ und R^{3'} für F und R^{4'} für H und R^{5'} für H oder
o) R^{2'} für F und R^{3'} für F und R^{4'} für H und R^{5'} für CF₃ oder
p) R^{2'} für CF₃ und R^{3'} für F und R^{4'} für H und R^{5'} für F steht.

Die Chinoloncarbonsäure-Derivate der Formel (V) können durch Umsetzungen mit Verbindungen der Formel (VI)

Z-H (VI)

in welcher
- Z: für Reste der Strukturen 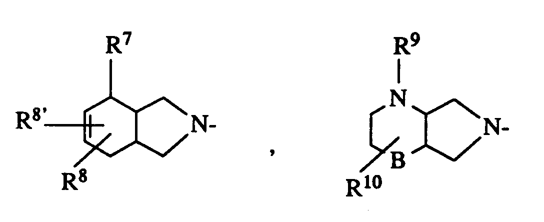
worin
- R⁷: für Wasserstoff, Hydroxy, -NR¹¹R¹², Hydroxymethyl oder -CH₂-NR¹¹R¹² steht,
wobei
- R¹¹: Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl und
- R¹²: Wasserstoff oder Methyl bedeutet,
- R⁸: für Wasserstoff, geradkettiges oder verzweigtes C₁-C₃-Alkyl oder Cyclopropyl,
- R^{8'}: für Wasserstoff oder Methyl,
- R⁹: für Wasserstoff oder Methyl,
- R¹⁰: für Wasserstoff, Methyl oder Reste der Strukturen -CH=CH-CO₂R^{9'}, -CH₂-CH₂-CO₂R^{9'}, -CH₂-CO-CH₃, -CH₂-CH₂-CN,
- R^{9'}: für Methyl oder Ethyl, und
- B: für -CH₂-, O oder eine direkte Bindung steht,
in Chinoloncarbonsäure-Derivate der Formel (VII) 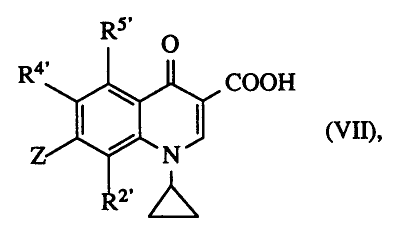 worin
- R^{2'}, R^{4'}, R^{5'}, R⁷ und Z: die oben angegebene Bedeutung haben,
überführt werden.

### Herstellungsbeispiele

### Beispiel 1

a) 1,3-Difluor-6-lithio-2-trifluormethylbenzol 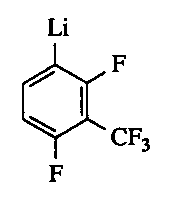
   Unter einer Stickstoff-Atmosphäre werden 0,5 mol 1,3-Difluor-2-trifluormethylbenzol in einem Gemisch aus 300 ml absolutem Tetrahydrofuran und 300 ml absolutem Diethylether gelöst und bei -70°C tropfenweise mit 220 ml (0,55 mol) einer 2,5 molaren n-Butyllithium-Lösung in Hexan versetzt und eine Stunde gerührt.
b) 2,4-Difluor-3-trifluormethylbenzoesäure 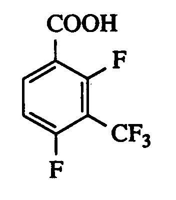
   Das gebildete 1,3-Difluor-6-lithio-2-trifluormethylbenzol wird ohne Zwischenisolierung zur 2,4-Difluor-3-trifluormethylbenzoesäure umgesetzt. Dazu werden in die gemäß 1a) erhaltene Lösung bei -70°C 200 g Kohlendioxid eingeleitet.
   Anschließend läßt man auf 5°C auftauen, rührt 1 Stunde nach und hydrolysiert mit 200 ml einer 2 N-Salzsäure. Die wässrige Phase wird abgetrennt und zweimal mit Diethylether extrahiert. Die vereinigten organischen Phasen werden mit verdünnter Salzsäure und mit Wasser gewaschen, über Magnesiumsulfat getrocknet und eingeengt.
   Es werden 98 g 2,4-Difluor-3-trifluormethylbenzoesäure vom Festpunkt 108-110°C erhalten, entsprechend einer Ausbeute von 86 %.

Analog zu Beispiel 1b) wurden die in Tabelle 1 aufgeführten Fluor-trifluormethylcarbonsäuren aus den entsprechenden lithiierten Verbindungen erhalten.

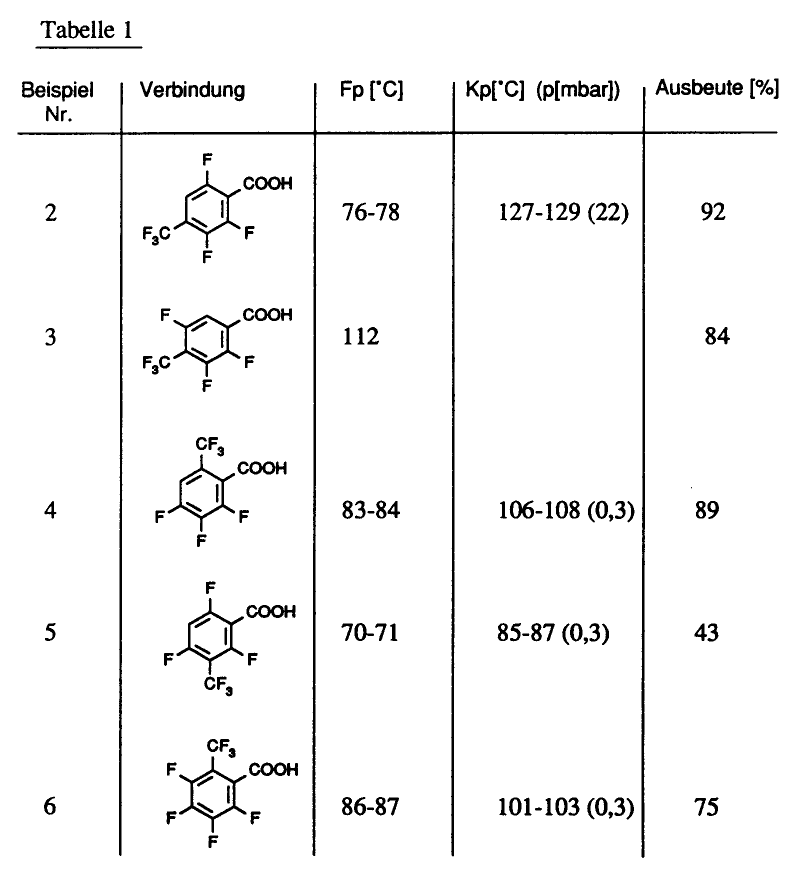

### Beispiel 7

### 2,4-Difluor-3-trifluormethylbenzoesäurechlorid

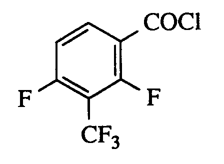

Zu 130 ml Thionylchlorid werden bei Raumtemperatur portionsweise 19,3 g (0,085 mol) 2,4-Difluor-3-trifluormethylbenzoesäure gemäß Beispiel 1 b) eingetragen. Nach dem Ende der Dosierung wird so lange auf 80°C erwärmt bis die Gasentwicklung beendet ist. Danach wird das überschüssige Thionylchlorid durch Destillation abgetrennt und das Rohprodukt direkt weiter umgesetzt.
Ausbeute: 20,0 g (90 % der Theorie)

### Beispiel 8

### 2,4,6-Trifluor-3-trifluormethylbenzoesäurechlorid

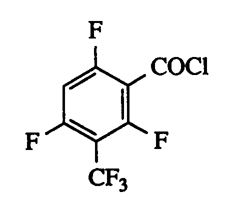

Zu 40 ml Thionylchlorid werden bei Raumtemperatur portionsweise 5,8 g (0,024 mol) 2,4,6-Trifluor-3-trifluormethylbenzoesäure gemäß Beispiel 5 eingetragen. Nach dem Ende der Dosierung wird so lange auf 80°C erwärmt bis die Gasentwicklung beendet ist. Danach wird das überschüssige Thionylchlorid durch Destillation abgetrennt und das Rohprodukt direkt weiter umgesetzt.
Ausbeute: 6,0 g (95 % der Theorie)

### Beispiel 9

### 2,3,4-Trifluor-6-trifluormethylbenzoesäurechlorid

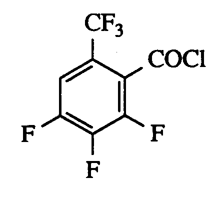

Zu 40 ml Thionylchlorid werden bei Raumtemperatur portionsweise 5,8 g (0,024 mol) 2,3,4-Trifluor-6-trifluormethylbenzoesäure gemäß Beispiel 4 eingetragen. Nach dem Ende der Dosierung wird so lange auf 80°C erwärmt bis die Gasentwicklung beendet ist. Danach wird das überschüssige Thionylchlorid durch Destillation abgetrennt und das Rohprodukt direkt weiter umgesetzt.
Ausbeute: 5,0 g (79 % der Theorie)

### Beispiel 10

### 1-Cyclopropyl-7-fluor-8-trifluormethyl-1,4-dihydro-4-oxo-3-chinolincarbonsäure

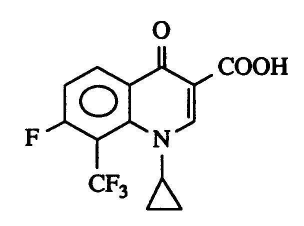

a) (2,4-Difluor-3-trifluormethylbenzoyl)malonsäurediethylester
   2,15 g (0,09 mol) Magnesiumspäne werden in 4,8 ml Ethanol vorgelegt, die Reaktion mit einigen Tropfen Tetrachlorkohlenstoff gestartet und anschließend die Lösung von 12,8 g (0,075 mol) Malonsäurediethylester in 9 ml Ethanol und 35 ml Toluol so zugetropft, daß die Innentemperatur zwischen 50 und 60°C liegt. Anschließend wird eine Stunde bei 60°C nachgerührt. Bei -10 bis -5°C wird die Lösung von 20,0 g (0,082 mol) 2,4-Difluor-3-trifluormethylbenzoesäurechlorid gemäß Beispiel 7 in 9 ml Toluol zugetropft, eine Stunde bei 0°C und anschließend unter Erwärmung auf Raumtemperatur nachgerührt. Der Ansatz wird auf 35 ml Eiswasser gegeben, mit 5,7 ml konzentrierter Schwefelsäure versetzt und mit Toluol extrahiert. Es wird mit gesättigter Natriumchloridlösung gewaschen und das Lösungsmittel im Vakuum entfernt.
   Rohausbeute: 30,0 g
b) (2,4-Difluor-3-trifluormethylbenzoyl)essigsäureethylester
   30,0 g des bei a) erhaltenen Rohproduktes werden in 30 ml Wasser mit 0,96 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.
   Rohausbeute: 22 g
c) 3-Ethoxy-2-(2,4-difluor-3-trifluormethylbenzoyl)-acrylsäureethylester
   22 g des unter b) erhaltenen Produktes werden mit 17,4 g (0,12 mol) Orthoameisensäureethylester und 19,4 g (0,19 mol) Essigsäureanhydrid zwei Stunden auf 150-160°C erwärmt. Überschüssiges Reagenz wird zunächst im Vakuum, dann im Hochvakuum bis zu einer Badtemperatur von 100°C entfernt.
   Rohausbeute: 23,1 g
d) 3-Cyclopropylamino-2-(2,4-difluor-4-trifluormethylbenzoyl)-acrylsäureethylester
   23,0 g (0,065 mol) des unter c) erhaltenen Produktes werden in 140 ml Ethanol bei 0°C vorgelegt und 4,08 g (0,072 mol) Cyclopropylamin zugetropft. Es wird über Nacht bei Raumtemperatur nachgerührt, 140 ml Wasser zugetropft und das ausfallende Produkt isoliert. Es wird mit Wasser nachgewaschen und bei ca. 100°C getrocknet.
   Ausbeute: 9,4 g (39 % der Theorie)
   Schmelzpunkt: 104-105°C
e) 1-Cyclopropyl-7-fluor-8-trifluormethyl-1,4-dihydro-4-oxo-3-chinolincarbonsäureethylester
   9,0 g (0,025 mol) des unter d) erhaltenen Produktes werden mit 3,9 g (0,028 mol) Kaliumcarbonat in 50 ml Dimethylformamid vier Stunden auf 100°C erwärmt. Der erkaltete Ansatz wird auf Eiswasser gegeben, das Produkt isoliert, mit Wasser nachgewaschen und bei 100°C getrocknet.
   Ausbeute: 8,1 g (98 % der Theorie)
   Schmelzpunkt: 154-155°C
f) 1-Cyclopropyl-7-fluor-8-trifluormethyl-1,4-dihydro-4-oxo-3-chinolincarbonsäure
   8,0 g (0,023 mol) des unter e) erhaltenen Produktes werden in einem Gemisch aus 30 ml Essigsäure, 30 ml Wasser und 3 ml konzentrierter Schwefelsäure zwei Stunden auf 140°C erwärmt. Der erkaltete Ansatz wird auf Eiswasser gegeben, das ausgefallene Produkt isoliert, mit Wasser nachgewaschen und bei 100°C getrocknet.
   Ausbeute: 7,0 g (96 % der Theorie)
   Schmelzpunkt: 209-210°C

### Beispiel 11

### 1-Cyclopropyl-5,7-difluor-8-trifluormethyl-1,4-dihydro-4-oxo-3-chinolincarbonsäure

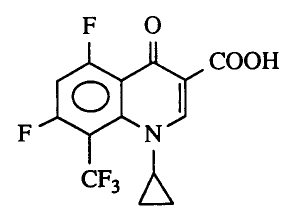

a) (2,4,6-Trifluor-3-trifluormethylbenzoyl)malonsäurediethylester
   0,58 g (0,024 mol) Magnesiumspäne werden in 1,3 ml Ethanol vorgelegt, die Reaktion mit einigen Tropfen Tetrachlorkohlenstoff gestartet und anschließend die Lösung von 3,4 g (0,019 mol) Malonsäurediethylester in 2,4 ml Ethanol und 9 ml Toluol so zugetropft, daß die Innentemperatur zwischen 50 und 60°C liegt. Anschließend wird eine Stunde bei 60°C nachgerührt. Bei -10 bis -5°C wird die Lösung von 5,8 g (0,027 mol) 2,4,6-Trifluor-3-trifluormethylbenzoesäurechlorid gemäß Beispiel 8 in 2,5 ml Toluol zugetropft, eine Stunde bei 0°C und anschließend unter Erwärmung auf Raumtemperatur nachgerührt. Der Ansatz wird auf 10 ml Eiswasser gegeben, mit 1,5 ml konzentrierter Schwefelsäure versetzt und mit Toluol extrahiert. Es wird mit gesättigter Natriumchloridlösung gewaschen und das Lösungsmittel im Vakuum entfernt.
   Rohausbeute: 8,6 g
b) (2,4,6-Trifluor-3-trifluormethylbenzoyl)essigsäureethylester
   8,6 g des bei a) erhaltenen Rohproduktes werden in 9 ml Wasser mit 0,26 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.
   Rohausbeute: 5,6 g
c) 3-Ethoxy-2-(2,4,6-trifluor-3-trifluormethylbenzoyl)-acrylsäureethylester
   5,4 g (0,017 mol) des unter b) erhaltenen Produktes werden mit 4,0 g (0,027 mol) Orthoameisensäureethylester und 4,45 g (0,043 mol) Essigsäureanhydrid zwei Stunden auf 150-160°C erwärmt. Überschüssiges Reagenz wird zunächst im Vakuum, dann im Hochvakuum bis zu einer Badtemperatur von 100°C entfernt.
   Rohausbeute: 3,8 g
d) 3-Cyclopropylamino-2-(2,4,6-trifluor-4-trifluormethylbenzoyl)-acrylsäureethylester
   3,8 g (0,01 mol) des unter c) erhaltenen Produktes werden in 22 ml Ethanol bei 0°C vorgelegt und 0,63 g (0,011 mol) Cyclopropylamin zugetropft. Es wird über Nacht bei Raumtemperatur nachgerührt, 22 ml Wasser zugetropft und das ausfallende Produkt isoliert. Es wird mit Wasser nachgewaschen und bei ca. 100°C getrocknet.
   Ausbeute: 3,3 g (86 % der Theorie)
   Schmelzpunkt: 146-148°C
e) 1-Cyclopropyl-5,7-difluor-8-trifluormethyl-1,4-dihydro-4-oxo-3-chinolincarbonsäureethylester
   3,5 g (9,2 mmol) des unter d) erhaltenen Produktes werden mit 1,45 g (0,01 mol) Kaliumcarbonat in 18 ml Dimethylformamid eine Stunde auf 100°C erwärmt. Der erkaltete Ansatz wird auf Eiswasser gegeben, das Produkt isoliert, mit Wasser nachgewaschen. Die Reinigung des Produktes erfolgt an Kieselgel Laufmittel Cyclohexan/Tetrahydrofuran 7/3.
   Ausbeute: 1,4 g (42 % der Theorie)
   Schmelzpunkt: 197-198°C
f) 1-Cyclopropyl-5,7-difluor-8-trifluormethyl-1,4-dihydro-4-oxo-3-chinolincarbonsäure
   1,4 g (3,9 mmol) des unter e) erhaltenen Produktes werden in einem Gemisch aus 5 ml Essigsäure, 5 ml Wasser und 0,5 ml konzentrierter Schwefelsäure zwei Stunden auf 140°C erwärmt. Der erkaltete Ansatz wird auf Eiswasser gegeben, das ausgefallene Produkt isoliert, mit Wasser nachgewaschen und bei 100°C getrocknet.
   Ausbeute: 1,1 g (84 % der Theorie)
   Schmelzpunkt: 208-210°C

### Beispiel 12

### 1-Cyclopropyl-7,8-difluor-5-trifluormethyl-1,4-dihydro-4-oxo-3-chinolincarbonsäure

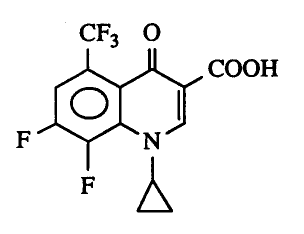

a) (2,3,4-Trifluor-6-trifluormethylbenzoyl)malonsäurediethylester
   0,47 g (0,019 mol) Magnesiumspäne werden in 1,0 ml Ethanol vorgelegt, die Reaktion mit einigen Tropfen Tetrachlorkohlenstoff gestartet und anschließend die Lösung von 2,8 g (0,016 mol) Malonsäurediethylester in 2,0 ml Ethanol und 7,5 ml Toluol so zugetropft, daß die Innentemperatur zwischen 50 und 60°C liegt. Anschließend wird eine Stunde bei 60°C nachgerührt. Bei -10 bis -5°C wird die Lösung von 4,8 g (0,027 mol) 2,3,4-Trifluor-6-trifluormethylbenzoesäurechlorid gemäß Beispiel 9 in 2,0 ml Toluol zugetropft, eine Stunde bei 0°C und anschließend unter Erwärmung auf Raumtemperatur nachgerührt. Der Ansatz wird auf 10 ml Eiswasser gegeben, mit 1,25 ml konzentrierter Schwefelsäure versetzt und mit Toluol extrahiert. Es wird mit gesättigter Natriumchloridlösung gewaschen und das Lösungsmittel im Vakuum entfernt.
   Rohausbeute: 6,6 g
b) (2,3,4-Trifluor-6-trifluormethylbenzoyl)essigsäureethylester
   6,6 g des bei a) erhaltenen Rohproduktes werden in 7,5 ml Wasser mit 0,21 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.
   Rohausbeute: 4,2 g
c) 3-Ethoxy-2-(2,3,4-trifluor-6-trifluormethylbenzoyl)-acrylsäureethylester
   4,0 g (0,013 mol) des unter b) erhaltenen Produktes werden mit 3,5 g (0,024 mol) Orthoameisensäureethylester und 3,4 g (0,033 mol) Essigsäureanhydrid zwei Stunden auf 150-160°C erwärmt. Überschüssiges Reagenz wird zunächst im Vakuum, dann im Hochvakuum bis zu einer Badtemperatur von 100°C entfernt.
   Rohausbeute: 2,7 g
d) 3-Cyclopropylamino-2-(2,3,4-trifluor-6-trifluormethylbenzoyl)-acrylsäureethylester
   2,7 g (7,3 mmol) des unter c) erhaltenen Produktes werden in 16 ml Ethanol bei 0°C vorgelegt und 0,46 g (8 mmol) Cyclopropylamin zugetropft. Es wird über Nacht bei Raumtemperatur nachgerührt, 16 ml Wasser zugetropft und das ausfallende Produkt isoliert. Es wird mit Wasser nachgewaschen und bei ca. 100°C getrocknet.
   Ausbeute: 2,1 g (75 % der Theorie)
   Schmelzpunkt: 165-168°C
e) 1-Cyclopropyl-7,8-difluor-5-trifluormethyl-1,4-dihydro-4-oxo-3-chinolincarbonsäureethylester
   2,1 g (5,5 mmol) des unter d) erhaltenen Produktes werden mit 0,88 g (6,4 mmol) Kaliumcarbonat in 11 ml Dimethylformamid eine Stunde auf 100°C erwärmt. Der erkaltete Ansatz wird auf Eiswasser gegeben, das Produkt isoliert, mit Wasser nachgewaschen.
   Ausbeute: 1,7 g (85 % der Theorie)
   Schmelzpunkt: 188-190°C
f) 1-Cyclopropyl-5,7-difluor-8-trifluormethyl-1,4-dihydro-4-oxo-3-chinolincarbonsäure
   1,5 g (4,2 mmol) des unter e) erhaltenen Produktes werden in einem Gemisch aus 5,5 ml Essigsäure, 5,5 ml Wasser und 0,55 ml konzentrierter Schwefelsäure zwei Stunden auf 140°C erwärmt. Der erkaltete Ansatz wird auf Eiswasser gegeben, das ausgefallene Produkt isoliert, mit Wasser nachgewaschen und bei 100°C getrocknet.
   Ausbeute: 1,3 g (92 % der Theorie)
   Schmelzpunkt: 226-228°C

### Beispiel 13

### 7-(4-Amino-1,3,3a,4,7,7a-hexahydroisoindol-2-yl)-1-cyclopropyl-8-fluor-5-trifluor methyl-1,4-dihydro-4-oxo-3-chinolincarbonsäure

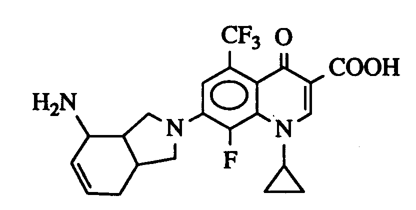

333 mg (1 mmol) 1-Cyclopropyl-7,8-difluor-5-trifluormethyl-1,4-dihydro-4-oxo-3-chinolincarbonsäure gemäß Beispiel 12 werden mit 165 mg (1,2 mmol) 4-Amino-1,3,3a,4,7,7a-hexahydroisoindol und 224 mg (2 mmol) 1,4-Diazabicyclo[2.2.2]octan in 10 ml Dimethylsulfoxid über Nacht bei Raumtemperatur gerührt. Alle flüchtigen Bestandteile werden im Hochvakuum entfernt, der Rückstand mit Acetonitril verrührt und bei 100°C getrocknet.
Ausbeute: 330 mg (73 % der Theorie)
Schmelzpunkt: 248-249°C (unter Zersetzung)

### Beispiel 14

### 7-(4-Amino-7-methyl-1,3,3a,4,7,7a-hexahydroisoindol-2-yl)-1-cyclopropyl-8-fluor-5-trifluormethyl-1,4-dihydro-4-oxo-3-chinolincarbonsäure

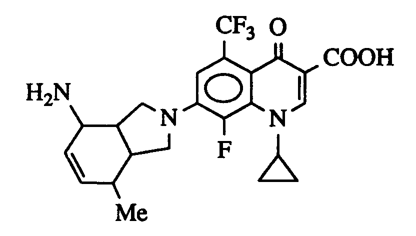

Analog zum Beispiel 13 wird bei der Umsetzung von 1-Cyclopropyl-7,8-difluor-5-trifluormethyl-1,4-dihydro-4-oxo-3-chinolincarbonsäure mit 4-Amino-7-methyl-1,3,3a,4,7,7a-hexahydroisoindol die Titelverbindung erhalten. Schmelzpunkt: 242-244°C (unter Zersetzung)

### Beispiel 15

### 7-(4-Amino-1,3,3a,4,7,7a-hexahydroisoindol-2-yl)-1-cyclopropyl-5-fluor-8-trifluormethyl-1,4-dihydro-4-oxo-3-chinolincarbonsäure

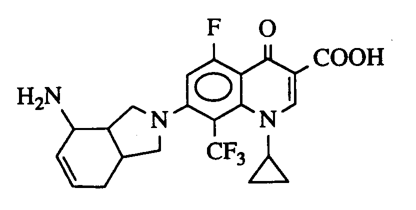

Analog zum Beispiel 13 wird bei der Umsetzung von 1-Cyclopropyl-5,7-difluor-8-trifluormethyl-1,4-dihydro-4-oxo-3-chinolincarbonsäure mit 4-Amino-7-methyl-1,3,3a,4,7,7a-hexahydroisoindol die Titelverbindung erhalten.
Schmelzpunkt: 238-240°C (unter Zersetzung)

### Beispiel 16

### 7-(4-Amino-7-methyl-1,3,3a,4,7,7a-hexahydroisoindol-2-yl)-1-cyclopropyl-5-fluor-8-trifluormethyl-1,4-dihydro-4-oxo-3-chinolincarbonsäure

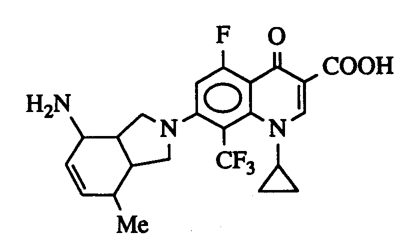

Analog zum Beispiel 13 wird bei der Umsetzung von 1-Cyclopropyl-5,7-difluor-8-trifluormethyl-1,4-dihydro-4-oxo-3-chinolincarbonsäure, erhalten gemäß Beispiel 11, mit 4-Amino-7-methyl-1,3,3a,4,7,7a-hexahydroisoindol die Titelverbindung erhalten.
Schmelzpunkt: 234-236°C (unter Zersetzung)

### Beispiel 17

### 7-(4-Amino-7-methyl-1,3,3a,4,7,7a-hexahydroisoindol-2-yl)-1-cyclopropyl-8-trifluormethyl-1,4-dihydro-4-oxo-3-chinolincarbonsäure

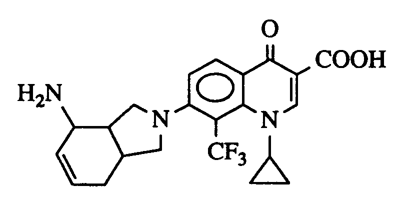

315 mg (1 mmol) 1-Cyclopropyl-7-fluor-8-trifluormethyl-1,4-dihydro-4-oxo-3-chinolincarbonsäure gemaß Beispiel 10 werden mit 165 mg (1,2 mmol) 4-Amino-1,3,3a,4,7,7a-hexahydroisoindol und 224 mg (2 mmol) 1,4-Diazabicyclo[2.2.2]octan in 10 ml Dimethylsulfoxid zwei Stunden bei 100°C gerührt. Alle flüchtigen Bestandteile werden im Hochvakuum entfernt, der Rückstand mit Acetonitril verrührt und bei 100°C getrocknet.
Ausbeute: 280 mg (65 % der Theorie)
Schmelzpunkt: 168-170°C (unter Zersetzung)

## Patentansprüche

1. Fluor-trifluormethylbenzoesäure-Derivate der Formel (I) 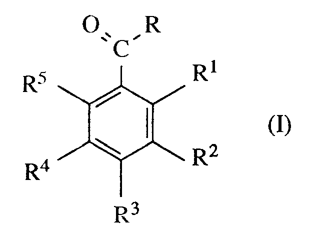 worin
R für Hydroxy, -OM, Halogen oder C₁-C₄-Alkoxy steht,
wobei
M für ein Alkalimetall, vorzugsweise für Lithium, Natrium oder Kalium steht,
und entweder
a) R¹ für F, R² für F, R³ für H, R⁴ für H und R⁵ für CF₃
oder b) R¹ für F, R² für CF₃, R³ für F, R⁴ für H und R⁵ für H
oder c) R¹ für F, R² für CF₃, R³ für H, R⁴ für H und R⁵ für F
oder d) R¹ für F, R² für F, R³ für CF₃, R⁴ für H und R⁵ für H
oder e) R¹ für F, R² für H, R³ für CF₃, R⁴ für H und R⁵ für F
oder f) R¹ für F, R² für F, R³ für F, R⁴ für H und R⁵ für CF₃
oder g) R¹ für F, R² für CF₃, R³ für F, R⁴ für H und R⁵ für F
oder h) R¹ für F, R² für F, R³ für H, R⁴ für CF₃ und R⁵ für F
oder i) R¹ für F, R² für F, R³ für F, R⁴ für CF₃ und R⁵ für H
oder k) R¹ für F, R² für F, R³ für CF₃, R⁴ für F und R⁵ für H
oder l) R¹ für F, R² für F, R³ für CF₃, R⁴ für H und R⁵ für F
oder m) R¹ für F, R² für F, R³ für F, R⁴ für F und R⁵ für CF₃
steht.

2. Verfahren zur Herstellung von Fluor-trifluormethylbenzoesäure-Derivaten gemäß Anspruch 1, entsprechend der Formel (I), mit R = Hydroxy dadurch gekennzeichnet, daß man Fluor-trifluormethylbenzole der Formel (IV) 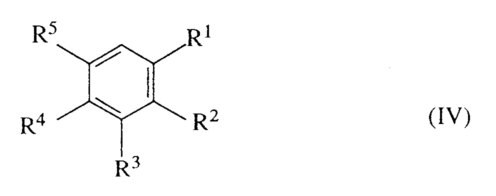 worin
entweder
a) R¹ für F, R² für F, R³ für H, R⁴ für H und R⁵ für CF₃
oder b) R¹ für F, R² für CF₃, R³ für F, R⁴ für H und R⁵ für H
oder c) R¹ für F, R² für CF₃, R³ für H, R⁴ für H und R⁵ für F
oder d) R¹ für F, R² für F, R³ für CF₃, R⁴ für H und R⁵ für H
oder e) R¹ für F, R² für H, R³ für CF₃, R⁴ für H und R⁵ für F
oder f) R¹ für F, R² für F, R³ für F, R⁴ für H und R⁵ für CF₃
oder g) R¹ für F, R² für CF₃, R³ für F, R⁴ für H und R⁵ für F
oder h) R¹ für F, R² für F, R³ für H, R⁴ für CF₃ und R⁵ für F
oder i) R¹ für F, R² für F, R³ für F, R⁴ für CF₃ und R⁵ für H
oder k) R¹ für F, R² für F, R³ für CF₃, R⁴ für F und R⁵ für H
oder l) R¹ für F, R² für F, R³ für CF₃, R⁴ für H und R⁵ für F
oder m) R¹ für F, R² für F, R³ für F, R⁴ für F und R⁵ für CF₃
steht,
mit einem Alkylmetallat, gegebenenfalls in Gegenwart eines Verdünnungsmittels bei einer Temperatur von -150 bis 30°C, vorzugsweise von -100 bis -20°C, umsetzt, so metallierte Fluor-trifluormethylbenzol-Derivate der Formel (II) erhält 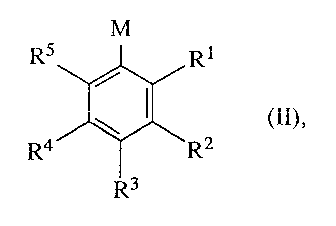 worin
M für ein Alkalimetall steht,
und R¹, R², R³, R⁴ und R⁵ die bei Formel (IV) angegebene Bedeutung haben,
die man mit Kohlendioxid, gegebenenfalls in Gegenwart eines Verdünnungsmittels bei einer Temperatur von -150 bis 50°C umsetzt und anschließend die gebildeten Alkalicarboxylate der Formel (I) gemäß Anspruch 1, mit R = OM, wobei M die in Anspruch 1 angegebene Bedeutung hat, mit einer Säure umsetzt.

3. Verfahren zur Herstellung von Fluor-trifluormethylhenzoesäure-Derivaten gemäß Anspruch 1, entsprechend der Formel (I) worin R für Halogen steht, dadurch gekennzeichnet, daß man Verbindungen der Formel (I) gemaß Anspruch 1 mit R = OH oder OM, mit einem Halogenierungsmittel, gegebenenfalls in Gegenwart eines Verdünnungsmittels bei einer Temperatur von 0 bis 160°C umsetzt.

4. Verfahren zur Herstellung von Fluor-trifluormethylbenzoesäure-Derivaten gemäß Anspruch 1, entsprechend der Formel (I), mit R = C₁-C₄-Alkoxy, dadurch gekennzeichnet, daß man Verbindungen der Formel (I) gemäß Anspruch 1, worin R für Hydroxy oder Halogen steht, mit einem Alkohol der Formel (III)
R⁶-OH (III)
worin
R⁶ für C₁-C₄-Alkyl steht,
gegebenenfalls in Gegenwart eines Verdünnungsmittels und gegebenenfalls in Gegenwart eines Säurebindemittels, bei einer Temperatur von 20 bis 160°C umsetzt.

5. Verbindungen der Formel (II) 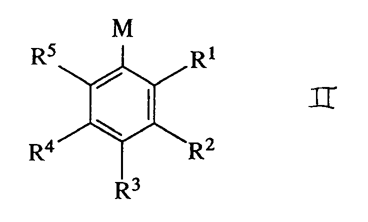 worin
M für ein Alkalimetall steht,
und entweder
a) R¹ für F, R² für F, R³ für H, R⁴ für H und R⁵ für CF₃
oder b) R¹ für F, R² für CF₃, R³ für F, R⁴ für H und R⁵ für H
oder c) R¹ für F, R² für CF₃, R³ für H, R⁴ für H und R⁵ für F
oder d) R¹ für F, R² für F, R³ für CF₃, R⁴ für H und R⁵ für H
oder e) R¹ für F, R² für H, R³ für CF₃, R⁴ für H und R⁵ für F
oder f) R¹ für F, R² für F, R³ für F, R⁴ für H und R⁵ für CF₃
oder g) R¹ für F, R² für CF₃, R³ für F, R⁴ für H und R⁵ für F
oder h) R¹ für F, R² für F, R³ für H, R⁴ für CF₃ und R⁵ für F
oder i) R¹ für F, R² für F, R³ für F, R⁴ für CF₃ und R⁵ für H
oder k) R¹ für F, R² für F, R³ für CF₃, R⁴ für F und R⁵ für H
oder l) R¹ für F, R² für F, R³ für CF₃, R⁴ für H und R⁵ für F
oder m) R¹ für F, R² für F, R³ für F, R⁴ für F und R⁵ für CF₃
steht.

## Claims

1. Fluoro-trifluoromethylbenzoic acid derivatives of the formula (I) 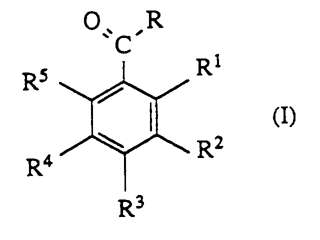 wherein
R represents hydroxyl, -OM, halogen or C₁-C₄-alkoxy,
wherein
M represents an alkali metal, preferably lithium, sodium or potassium,
and either
a) R¹ represents F, R² represents F, R³ represents H, R⁴ represents H and R⁵ represents CF₃
or b) R¹ represents F, R² represents CF₃, R³ represents F, R⁴ represents H and R⁵ represents H
or c) R¹ represents F, R² represents CF₃, R³ represents H, R⁴ represents H and R⁵ represents F
or d) R¹ represents F, R² represents F, R³ represents CF₃, R⁴ represents H and R⁵ represents H
or e) R¹ represents F, R² represents H, R³ represents CF₃, R⁴ represents H and R⁵ represents F
or f) R¹ represents F, R² represents F, R³ represents F, R⁴ represents H and R⁵ represents CF₃
or g) R¹ represents F, R² represents CF₃, R³ represents F, R⁴ represents H and R⁵ represents F
or h) R¹ represents F, R² represents F, R³ represents H, R⁴ represents CF₃ and R⁵ represents F
or i) R¹ represents F, R² represents F, R³ represents F, R⁴ represents CF₃ and R⁵ represents H
or k) R¹ represents F, R² represents F, R³ represents CF₃, R⁴ represents F and R⁵ represents H
or l ) R¹ represents F, R² represents F, R³ represents CF₃, R⁴ represents H and R⁵ represents F
or m) R¹ represents F, R² represents F, R³ represents F, R⁴ represents H and R⁵ represents CF₃.

2. Process for the preparation of fluoro-trifluoromethylbenzoic acid derivatives according to Claim 1, corresponding to the formula (I), where R = hydroxyl, characterized in that fluoro-trifluoromethylbenzenes of the formula (IV) 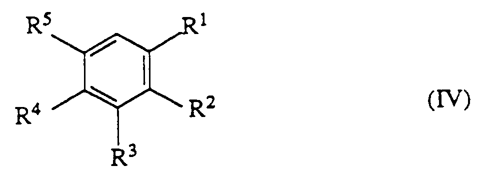 wherein
either
a) R¹ represents F, R² represents F, R³ represents H, R⁴ represents H and R⁵ represents CF₃
or b) R¹ represents F, R² represents CF₃, R³ represents F, R⁴ represents H and R⁵ represents H
or c) R¹ represents F, R² represents CF₃, R³ represents H, R⁴ represents H and R⁵ represents F
or d) R¹ represents F, R² represents F, R³ represents CF₃, R⁴ represents H and R⁵ represents H
or e) R¹ represents F, R² represents H, R³ represents CF₃, R⁴ represents H and R⁵ represents F
or f) R¹ represents F, R² represents F, R³ represents F, R⁴ represents H and R⁵ represents CF₃
or g) R¹ represents F, R² represents CF₃, R³ represents F, R⁴ represents H and R⁵ represents F
or h) R¹ represents F, R² represents F, R³ represents H, R⁴ represents CF₃ and R⁵ represents F
or i) R¹ represents F, R² represents F, R³ represents F, R⁴ represents CF₃ and R⁵ represents H
or k) R¹ represents F, R² represents F, R³ represents CF₃, R⁴ represents F and R⁵ represents H
or l) R¹ represents F, R² represents F, R³ represents CF₃, R⁴ represents H and R⁵ represents F
or m) R¹ represents F, R² represents F, R³ represents F, R⁴ represents H and R⁵ represents CF₃,
are reacted with an alkyl metallate, optionally in the presence of a diluent, at a temperature of -150 to 30°C, preferably of -100 to -20°C, thus giving metallized fluoro-trifluoromethylbenzene derivatives of the formula (II) 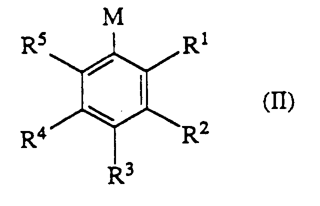 wherein
M represents an alkali metal,
and R¹, R², R³, R⁴ and R⁵ have the meaning given in formula (IV),
which are reacted with carbon dioxide, if appropriate in the presence of a diluent, at a temperature of -150 to 50°C and subsequently the alkali metal carboxylates of the formula (I) according to Claim 1 formed, where R = OM, wherein M has the meaning given in Claim 1, are reacted with an acid.

3. Process for the preparation of fluoro-trifluoromethylbenzoic acid derivatives according to Claim 1, corresponding to the formula (I) wherein R represents halogen, characterized in that compounds of the formula (I) according to Claim 1 where R = OH or OM, are reacted with a halogenating agent, if appropriate in the presence of a diluent, at a temperature of 0 to 160°C.

4. Process for the preparation of fluoro-trifluoromethylbenzoic acid derivatives according to Claim 1, corresponding to the formula (I) where R = C₁-C₄-alkoxy, characterized in that compounds of the formula (I) according to Claim 1 wherein R represents hydroxyl or halogen are reacted with an alcohol of the formula (III)
R⁶-OH (III)
wherein
R⁶ represents C₁-C₄-alkyl,
if appropriate in the presence of a diluent and if appropriate in the presence of an acid-binding agent, at a temperature of 20 to 160°C.

5. Compounds of the formula (II) 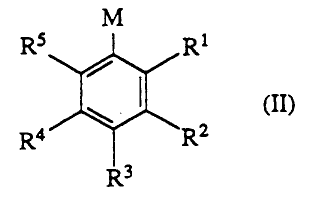 wherein
M represents an alkali metal,
and either
a) R¹ represents F, R² represents F, R³ represents H, R⁴ represents H and R⁵ represents CF₃
or b) R¹ represents F, R² represents CF₃, R³ represents F, R⁴ represents H and R⁵ represents H
or c) R¹ represents F, R² represents CF₃, R³ represents H, R⁴ represents H and R⁵ represents F
or d) R¹ represents F, R² represents F, R³ represents CF₃, R⁴ represents H and R⁵ represents H
or e) R¹ represents F, R² represents H, R³ represents CF₃, R⁴ represents H and R⁵ represents F
or f) R¹ represents F, R² represents F, R³ represents F, R⁴ represents H and R⁵ represents CF₃
or g) R¹ represents F, R² represents CF₃, R³ represents F, R⁴ represents H and R⁵ represents F
or h) R¹ represents F, R² represents F, R³ represents H, R⁴ represents CF₃ and R⁵ represents F
or i) R¹ represents F, R² represents F, R³ represents F, R⁴ represents CF₃ and R⁵ represents H
or k) R¹ represents F, R² represents F, R³ represents CF₃, R⁴ represents F and R⁵ represents H
or l) R¹ represents F, R² represents F, R³ represents CF₃, R⁴ represents H and R⁵ represents F
or m) R¹ represents F, R² represents F, R³ represents F, R⁴ represents H and R⁵ represents CF₃.

## Revendications

1. Dérivés d'acides fluoro-trifluorométhylbenzoïques de formule (I) 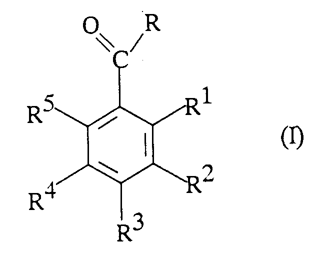 dans laquelle
R représente un groupe hydroxy, -OM, un halogène ou un groupe alcoxy en C₁-C₄,
M représentant un métal alcalin, de préférence le lithium, le sodium ou le potassium,
et
a) R¹ représente F, R² représente F, R³ représente H, R⁴ représente H et R⁵ représente CF₃
ou bien b) R¹ représente F, R² représente CF₃, R³ représente F, R⁴ représente H et R⁵ représente H
ou bien c) R¹ représente F, R² représente CF₃, R³ représente H, R⁴ représente H et R⁵ représente F
ou bien d) R¹ représente F, R² représente F, R³ représente CF₃, R⁴ représente H et R⁵ représente H
ou bien e) R¹ représente F, R² représente H, R³ représente CF₃, R⁴ représente H et R⁵ représente F
ou bien f) R¹ représente F, R² représente F, R³ représente F, R⁴ représente H et R⁵ représente CF₃
ou bien g) R¹ représente F, R² représente CF₃, R³ représente F, R⁴ représente H et R⁵ représente F
ou bien h) R¹ représente F, R² représente F, R³ représente H, R⁴ représente CF₃ et R⁵ représente F
ou bien i) R¹ représente F, R² représente F, R³ représente F, R⁴ représente CF₃ et R⁵ représente H
ou bien k) R¹ représente F, R² représente F, R³ représente CF₃, R⁴ représente F et R⁵ représente H
ou bien l) R¹ représente F, R² représente F, R³ représente CF₃, R⁴ représente H et R⁵ représente F
ou bien m) R¹ représente F, R² représente F, R³ représente F, R⁴ représente F et R⁵ représente CF₃.

2. Procédé de préparation des dérivés d'acides fluoro-trifluorométhylbenzoïques selon la revendication 1, répondant à la formule (I), dans laquelle R représente un groupe hydroxy, caractérisé en ce que l'on fait réagir des fluoro-trifluorométhylbenzènes de formule (IV) 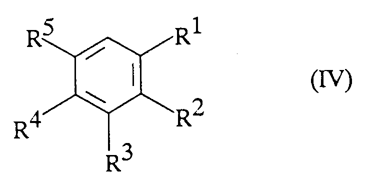 dans laquelle
a) R¹ représente F, R² représente F, R³ représente H, R⁴ représente H et R⁵ représente CF₃
ou bien b) R¹ représente F, R² représente CF₃, R³ représente F, R⁴ représente H et R⁵ représente H
ou bien c) R¹ représente F, R² représente CF₃, R³ représente H, R⁴ représente H et R⁵ représente F
ou bien d) R¹ représente F, R² représente F, R³ représente CF₃, R⁴ représente H et R⁵ représente H
ou bien e) R¹ représente F, R² représente H, R³ représente CF₃, R⁴ représente H et R⁵ représente F
ou bien f) R¹ représente F, R² représente F, R³ représente F, R⁴ représente H et R⁵ représente CF₃
ou bien g) R¹ représente F, R² représente CF₃, R³ représente F, R⁴ représente H et R⁵ représente F
ou bien h) R¹ représente F, R² représente F, R³ représente H, R⁴ représente CF₃ et R⁵ représente F
ou bien i) R¹ représente F, R² représente F, R³ représente F, R⁴ représente CF₃ et R⁵ représente H
ou bien k) R¹ représente F, R² représente F, R³ représente CF₃, R⁴ représente F et R⁵ représente H
ou bien l) R¹ représente F, R² représente F, R³ représente CF₃, R⁴ représente H et R⁵ représente F
ou bien m) R¹ représente F, R² représente F, R³ représente F, R⁴ représente F et R⁵ représente CF₃,
avec un alkylmétallate, éventuellement en présence d'un diluant, à des températures allant de -150 à +30°C, de préférence de -100 à -20°C, ce qui donne des dérivés métallisés de fluoro-trifluorométhylbenzènes de formule (II) 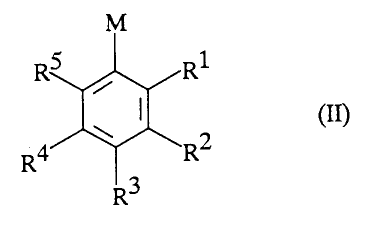 dans laquelle
M représente un métal alcalin et
R¹, R², R³, R⁴ et R⁵ ont les significations indiquées en référence à la formule (IV), qu'on fait réagir avec le dioxyde de carbone, éventuellement en présence d'un diluant, à des températures allant de -150 à +50°C, ce qui donne des carboxylates alcalins de formule (I) selon revendication 1, dans laquelle R = OM, M ayant les significations indiquées dans la revendication 1, qu'on fait réagir avec un acide.

3. Procédé de préparation des dérivés d'acides fluoro-trifluorométhylbenzoïques selon revendication 1, répondant à la formule (I) dans laquelle R représente un halogène, caractérisé en ce que l'on fait réagir des composés de formule (I) selon revendication 1 dans laquelle R = OH ou OM, avec un agent halogénant, éventuellement en présence d'un diluant, à des températures de 0 à 160°C.

4. Procédé de préparation des dérivés d'acides fluoro-trifluorométhylbenzoïques selon revendication 1, répondant à la formule (I) dans laquelle R représente un groupe alcoxy en C₁-C₄, caractérisé en ce que l'on fait réagir des composés de formule (I) selon revendication 1, dans laquelle R représente un groupe hydroxy ou un halogène, avec un alcool de formule (III)
R⁶-OH (III)
dans laquelle
R⁶ représente un groupe alkyle en C₁-C₄,
éventuellement en présence d'un diluant et éventuellement en présence d'un capteur d'acide, à des températures allant de 20 à 160°C.

5. Composés de formule (II) 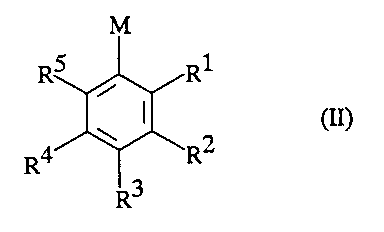 dans laquelle
M représente un métal alcalin et
a) R¹ représente F, R² représente F, R³ représente H, R⁴ représente H et R⁵ représente CF₃
ou bien b) R¹ représente F, R² représente CF₃, R³ représente F, R⁴ représente H et R⁵ représente H
ou bien c) R¹ représente F, R² représente CF₃, R³ représente H, R⁴ représente H et R⁵ représente F
ou bien d) R¹ représente F, R² représente F, R³ représente CF₃, R⁴ représente H et R⁵ représente H
ou bien e) R¹ représente F, R² représente H, R³ représente CF₃, R⁴ représente H et R⁵ représente F
ou bien f) R¹ représente F, R² représente F, R³ représente F, R⁴ représente H et R⁵ représente CF₃
ou bien g) R¹ représente F, R² représente CF₃, R³ représente F, R⁴ représente H et R⁵ représente F
ou bien h) R¹ représente F, R² représente F, R³ représente H, R⁴ représente CF₃ et R⁵ représente F
ou bien i) R¹ représente F, R² représente F, R³ représente F, R⁴ représente CF₃ et R⁵ représente H
ou bien k) R¹ représente F, R² représente F, R³ représente CF₃, R⁴ représente F et R⁵ représente H
ou bien l) R¹ représente F, R² représente F, R³ représente CF₃, R⁴ représente H et R⁵ représente F
ou bien m) R¹ représente F, R² représente F, R³ représente F, R⁴ représente F et R⁵ représente CF₃.
